# EUROPEAN PATENT APPLICATION

(11) **EP 3 332 703 A1**
(43) Date of publication of application: **13.06.2018**
(21) Application number: 17204937.1
(22) Date of filing: 01.12.2017
(51) Int. Cl.: A61B 5/04, A61B 17/34

(54) **A SURGICAL INSERTION DEVICE**

(30) Priority: 08.12.2016 EP 16202828 P
(71) Applicant: IMEC vzw, 3001 Leuven (BE); Universiteit Gent, 9000 Gent (BE)
(72) Inventor: Op de Beeck, Maria, 3001 Leuven (BE); Vandecasteele, Bjorn, 3001 Leuven (BE); Braeken, Dries, 3001 Leuven (BE)
(74) Representative: Patent Department IMEC

(57) **Abstract**

A surgical insertion device (1) is disclosed, comprising an elongated central flat region (2) tapering from the proximal end having a device area (4), configured to receive an electronic device (5), towards the distal end having a sharp tip (3).

## Description

### Field

The present disclosure relates to surgical insertion devices for implanting an electronic device into body tissue. This electronic device comprises Integrated Circuits, Micro-Electro-Mechanical Systems (MEMS), interconnect boards or a combination thereof. Typically these electronic devices are sensory devices.

### Background

When devices are implanted in body tissue, it is important to perform this implantation with minimal damage to the tissue. Damaged tissue will lead to more difficult wound healing and more scar tissue development. Avoiding tissue damage upon device implantation is especially important if this device contains sensors which need to be in direct contact with the local tissue. If the tissue adjacent to the sensors is damaged, formation of neighboring scar tissue may limit, e.g. the registration of signals by the sensors.

Current procedures for implanting devices, e.g. sensory device, into body tissue take this effect into account. Hence, surgeons will avoid making a large incision and will just insert the device into the body tissue.
An adjusted procedure can be used. A small incision is made and a guiding tool is used to position the sensory device into the body tissue. Typically the to-be-implanted device is located inside this guiding tool. When the guiding tool is at the correct position in the body tissue, the to-be-implanted device is pushed out of the guiding tool. After placement of the device in the body tissue, the guiding tool is removed from the body tissue. Since the guiding tool is larger than the to-be-implanted device, the incision made is often slightly larger than the size of the implant device. Moreover, it might prove difficult to precisely positioning the to-be-implanted device in the body tissue by using a guiding tool. Furthermore, not all implant devices are suitable for this an implantation procedure.
Another procedure for insertion of an implant device uses an implant device having attached to it a surgical suture with a needle. First the needle is inserted into the body tissue at the desired location and then pushed through the body tissue. Using the suture, the implant device is pulled into the body tissue until the implant device is positioned at the predetermined location in the body tissue. Then the suture with needle is cut and removed from the device implant. This procedure is particularly suited for narrow, but long, cylindrical shaped implant devices. Such implant devices are preferably made conical to allow a smoother insertion into the body tissue when pulling at the suture. However, pulling the implant device into the body tissue may cause some tissue damage, as the implant device itself is typically substantially larger than the needle. Furthermore, not all implant devices are fit for insertion using this implant procedure. Depending on the implant device application, when implanted, the implant device must be oriented towards selected tissue to ensure correct sensing thereof. However, this implantation technique doesn't allow precisely controlling the orientation of the implant device, when inserting the implant device into the body tissue.

Hence, there is a need for an surgical insertion device reducing the damage to body tissue when inserting an implant device. Preferably this insertion device allows inserting implant devices with arbitrarily shape. Furthermore, this insertion device provides an improved control over the orientation and/or position of the implant device when in the body tissue.

### Drawings

The present disclosure will be described with respect to particular embodiments and with reference to certain drawings but the disclosure is not limited thereto but only by the claims. The drawings described are only schematic and are non-limiting. In the drawings, the size of some of the elements maybe exaggerated and not drawn on scale for illustrative purposes. The dimensions and the relative dimensions do not correspond to actual reductions to practice of the disclosure. In the different figures, the same reference signs refer to the same or analogous elements.
FIG. 1 illustrates a top view of a surgical insertion device according to this disclosure.
FIG. 2a-b illustrate top views of surgical insertion devices according to this disclosure.
FIG. 3a-e illustrate by top views a method for inserting an implant device into body tissue using a surgical device according to this disclosure.
FIG. 4a-c illustrate by top views another method for inserting an implant device into body tissue using a surgical device according to this disclosure.

### Summary

A surgical insertion device, comprising an elongated central flat region tapering from one distal end having a device area, configured to receive an electronic device, towards the opposite distal end having a sharp tip. The central flat region (2), the sharp tip (3) and the device area (4) can be integral with each other. Alternatively, the sharp tip (3) and or the device area (4) is attached to the central flat region (2). Preferably, the insertion device (1) has at least one opening (6) adjacent to the device area (4). Optionally, barbs (7) can be present, at least at one side, near the maximal with (W2) of the insertion device (1), i.e. near the device area (4) or device holder (4). An electronic device (5) is attached to it at the distal end opposite to the sharp tip (3), in particular when in use. This electronic device (5) can be embedded in the insertion device (1) e.g. by means of packaging the electronic device (5). This electronic device (5) can be at least one electrode (10), electrically connected to another electronic device (10), positioned more remote from the sharp tip (3).

### Description

Moreover, the terms top, bottom, over, under and the like in the description and the claims are used for descriptive purposes and not necessarily for describing relative positions. It is to be understood that the terms so used are interchangeable under appropriate circumstances and that the embodiments of the disclosure described herein are capable of operation in other orientations than described or illustrated herein.
It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present disclosure, the only relevant components of the device are A and B.
Reference throughout this specification to "one embodiment" or "an embodiment" means that a particular feature, structure or characteristic described in connection with the embodiment is included in at least one embodiment of the present disclosure. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments. Similarly it should be appreciated that in the description of exemplary embodiments of the disclosure, various features of the disclosure are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed disclosure requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this disclosure.
Furthermore, while some embodiments described herein include some but not other features included in other embodiments, combinations of features of different embodiments are meant to be within the scope of the disclosure, and form different embodiments, as would be understood by those in the art. For example, in the following claims, any of the claimed embodiments can be used in any combination.

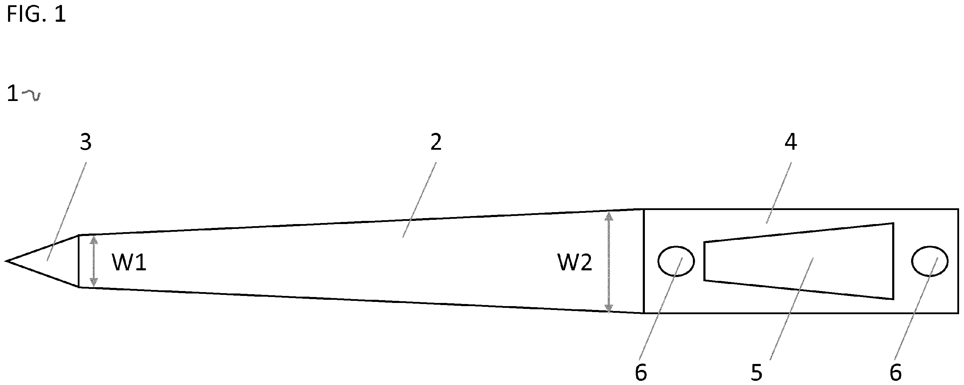

As illustrated by FIG. 1, a surgical insertion device (1) is disclosed comprising at one distal end a sharp tip (3) which act as a needle or knife making an incision in the body tissue. At the other distal end a device area (4) is provided, configured to receive the device (5) to-be-implanted. The device area (4) is thus an electronic device holder. Both ends (3, 4) are connected via a center part or ribbon (2) having a width gradually varying from the width W1 of the sharp tip (3) to the width W2 of the device area (4). This gradual transition in width assists in smootly widening the incision made by the sharp tip (3), when further inserting the surgical insertion device (1) in the body tissue, until the width of the to-be-implanted device (5) is reached. If barbs (7) are present at the side of the insertion device (1) where it is widest, typically near the the to-be-implanted device (5), it is benificial that the incision in the body tissue is smaller as then these barbs (7) will have a better grip to the body tissue. Hence the width of the insertion device (1) at the location of the to-be-implanted device (5) should only be slightly larger than the width of the to-be-implanted device (5). As the final opening of the incision in the body tissue is determined by the maximal width of the insertion device (1), the to-be-implanted device (5) can be an arbitrary shape: it need not be elongated, conical or rectangular. The thickness of the insertion device (1) is small compared to its width, resulting in an elongated flat device (1). Hence, the insertion device (1) may be paper-knife shaped.

Although the surgical insertion device (1) can be manufactured integrally, whereby the sharp tip (3), the center part or ribbon (2) and the device area (4) are formed together, the disclosure is not limited thereto. Instead of having the insertion device (1) formed as one piece, one can also assembly the insertion device (1) by attaching to the center part (2) made of a first material or set of material(s), the sharp tip (3) made of a second material or second set of material(s) selected to provide to provide the required sharpness and mechanical strength exerted upon the insertion device (1) when pushing the tip through the body tissue. The device area (4) can be formed of this first material, e.g. formed integral with the central part (2) or, when formed from another material or set of material(s), can be attached to the center part (2). In the latter case it may be easier to remove, after insertion, that part (2, 3) of the insertion device (1) that is not supporting the to-implanted-device (5).
One can also embed the to-be-implanted device (5) in the material(s) forming the insertion device (1). One can position the to-be-implanted device (5) on the device area (4) and then apply a material covering this device (5). As an alternative, the layers used to package the to-be-implanted device (5) can be patterned to extend in sideward direction, thereby forming the device area (4), and in lateral direction, thereby forming the center part (2), optionally also forming the sharp tip (3).

One or more openings (6) can be provided in the surgical insertion device (1), preferably adjacent to location where the to-be-implanted device will be attached to the insertion device (1). These openings can be used to secure the part of the insertion device (1), containing the to-be-implanted device (5), to the tissue, e.g. by using surgical suture.

The device-to-be-implanted (5) can be an Integrated Circuit, a Micro-Electro-Mechanical Systems (MEMS), an interconnect board or a combination thereof. Typically these electronic devices are sensory devices. Such a device (5) can e.g. be a board containing a metal pattern with functionalized electrodes interacting with the body tissue and/or body liquids to be examined, whereby the metal pattern allows accessing these electrodes and providing an interconnect to other electronic systems (10) in the body tissue.

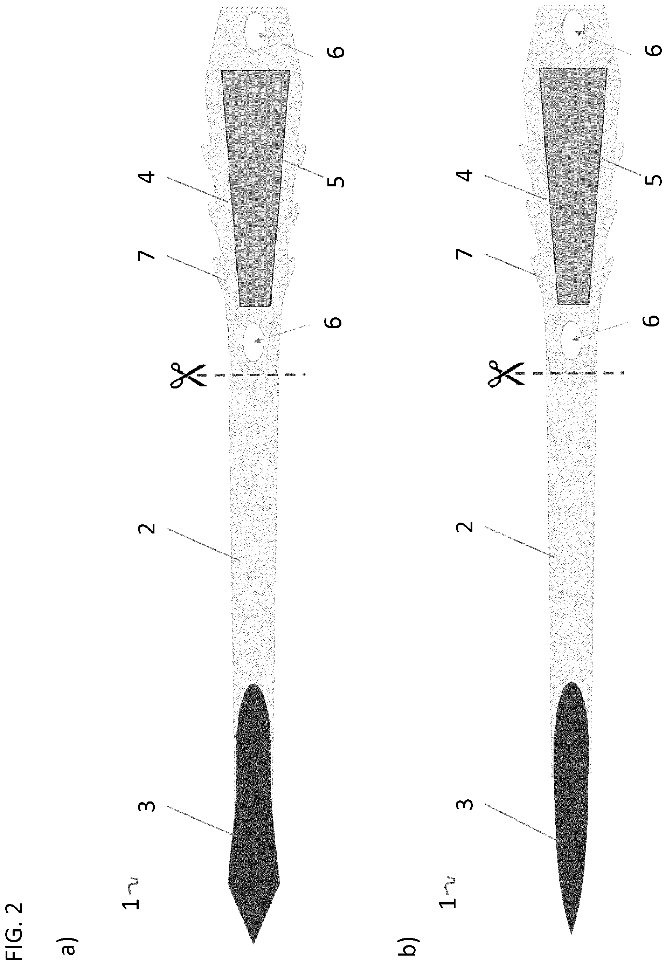

FIG. 2a and FIG. 2b shows preferred embodiments of such surgical insertion device (1). Attached to the center part or guiding ribbon (2) is a sharp tip (3). The guiding ribbon (2) extends in lateral direction from the tip (3) to form a device area (4) to which the to-be-implanted device (5) is or can be attached. The center part may taper from the device area (4), having a width determined by the width of the to-be-implanted device (5), towards the sharp tip (3). Openings (6) are present in the ribbon at opposite sides of the to-be-implanted device (5). The longitudinal sides of the device area (4) have barbs (7) allowing sliding the insertion device (1) in the body tissue, but the insertion device (1) will then be hooked in the body tissue preventing further movements of the implant device (5) relative to the body tissue. In FIG. 2a the tip (3) has a triangular-shaped end. In FIG. 2b the tip (3) has a oval-shaped end.

This insertion device (1) is particularly suitable for flat devices (5), which need maximal contact with local tissue. The procedure consists of insertion the device (5) by pulling at a thin, flat implantation ribbon (2). This ribbon (2) is attached, e.g. glued, at one side to the to-be-implanted device (5). Alternatively the ribbon (3) is fabricated as part of the to-be-implanted device (5). The other part of the ribbon (2) is attached to a miniaturized 'knife/needle' structure (3). This ribbon (2) can be made from a strong biocompatible polymer such as poly-imide. The knife/needle (3) is a very thin hard piece with a triangular or arrow like shape, made from a hard material such as SST (stainless steel), in order to allow to cut through tissue accurately with minimal tissue damage. The knife/needle (3) has a sharp point at its tip and is manipulated using a pair of tweezers or a conventional surgical needle holder in order to allow puncturing of the tissue at the most ideal location for the implant. After pushing the knife/needle (3) through the tissue, the ribbon (2) attached to the knife/needle (3) can be pulled through the tissue until the device (5) is located in its optimum position. The ribbon (2) is flat and will pass through the tissue in the same orientation as the knife/needle (3), and hence the device (5) will be inserted into the tissue in exact the same and hence well controlled orientation as of the ribbon (2). Especially when the device (5) is flat, this insertion will take place with minimum tissue damage, since the knife/needle (3) and ribbon (2) are all flat and pass through the tissue in the same way. If the device (5), which is implanted is larger than the ribbon (2) and knife/needle (3), then this device (5) has preferably a triangular shape having its tip pointing towards the knife/needle structure (3) to allow a smooth insertion into the incision in the body tissue made by the knife/needle (3). The implantation ribbon (2) can be attached to the device (5) after fabrication of this device (5), or can be fabricated simultaneously with the device (5) when shaping the polymers which are encapsulating the device (5). All used materials should be selected with the required biocompatibility properties for this medical application. After implantation in the body tissue, the device (5) needs to remain at its optimum position. This can be realized by using a small saw tooth profile fabricated at the sides of the insertion device (1) adjacent to the device (5). The saw tooth profile should be designed to allow for easy insertion, but to stop a possible sliding back of the implant device (5) after the insertion and positioning. An additional suture at the tip of the implant device (5) can be used to guarantee a stable long term implant positioning. After this procedure, the guiding ribbon (2) can be cut and removed together with the tip of the insertion device (1), thereby leaving the implant device (5) and a small corresponding part of the insertion device (1) in the body tissue.

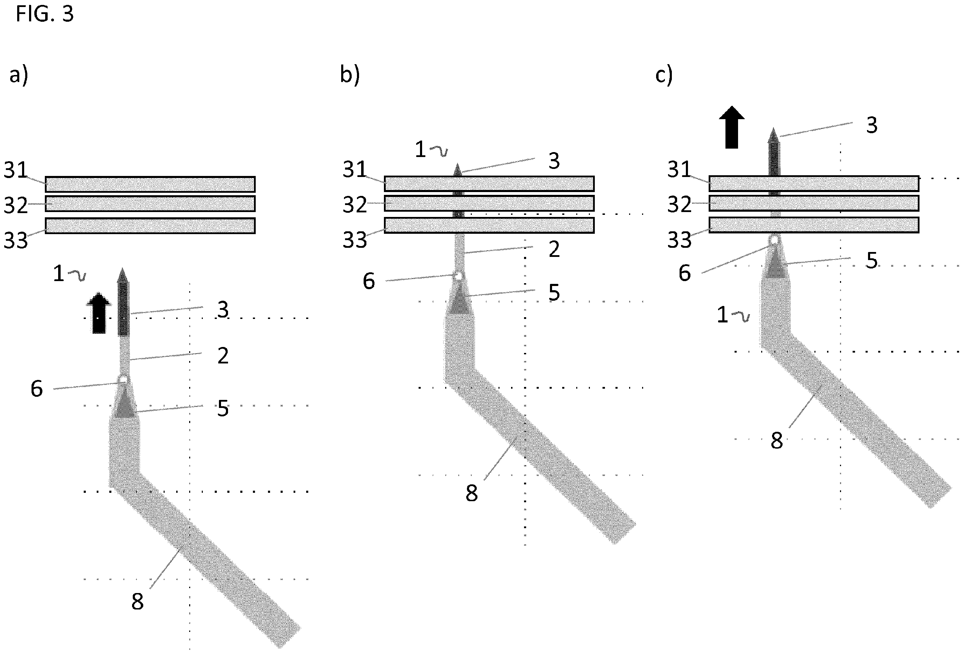

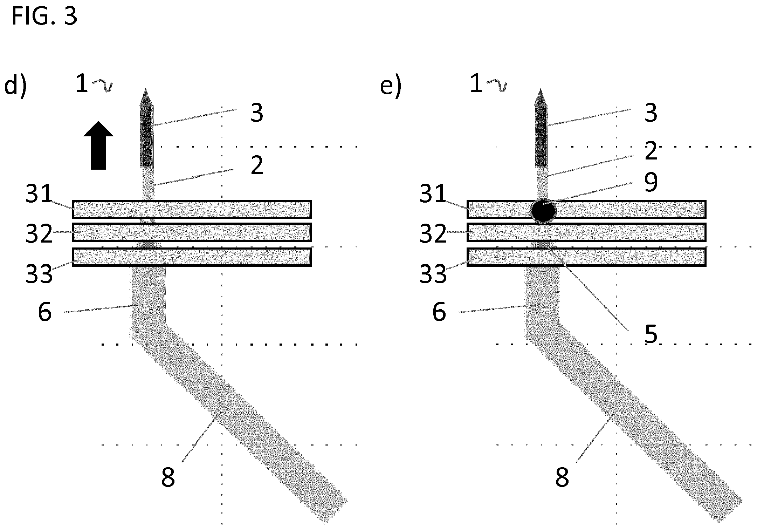

FIG. 3a-e illustrate a surgical insertion procedure using a surgical insertion device according to this disclosure. For the purpose of teaching, a chip (5) is inserted into a nerve bundle (31, 32, 33). The chip (5) is packaged into polymers. As illustrated in FIG. 2, these polymers are shaped in a saw tooth shaped profile near the sloped device edges to allow for stable fixation of the implanted device after insertion thereof in the tissue (31, 32, 33). FIG. 3a illustrates how the surgical insertion device (1), containing at one distal end the to-be-implanted device (5), is to be brought into contact with the tissue (31, 32, 33). A surgical needle holder (8) is used to manipulate the insertion device (1). FIG. 3b illustrates how the insertion device (3) is pushed into the tissue (31, 32, 33) with the sharp end first until its tip (3) becomes visible at the opposite side of the tissue (31,32,33). As illustrated in FIG. 3c the tip is taken by, e.g. a pair of tweezers, and by pulling at the tip, the insertion device (1) will slide further, well controlled, through the tissue (31, 32, 33), i.e. at the predetermined insertion position along the predetermined orientation. As illustrated in FIG. 3d, one continues pulling at the insertion device until the device (5) is at the desired position with regard to the tissue (31, 32, 33). As illustrated in FIG. 3e, once the to-be-implanted device (5) is at this desired position, it will remain fixed thanks to the saw tooth sides (7) of the insertion device (1). Additionally, the part (4) of the insertion device (1) containing the implant device (5) can be further fixed by a suture (9) through the tissue (31, 32, 33) and the fixation hole (6). If desired, the other part (2) of the insertion device (1) can be cut off thereby leaving the chip (5) in the tissue (31, 32, 33).

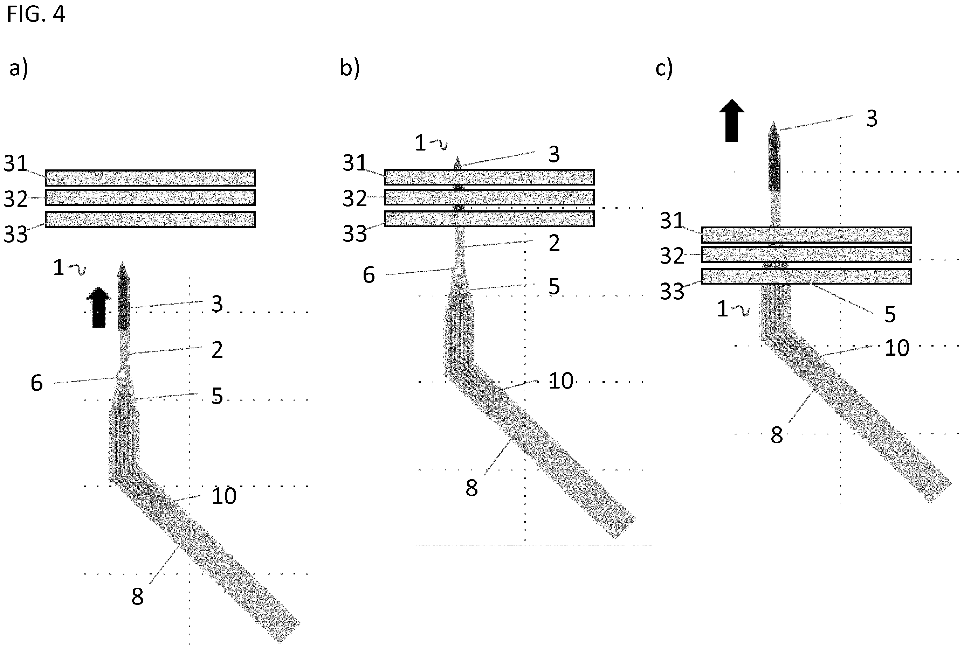

FIG. 4a-c FIG. 3a-e illustrate another surgical insertion procedure using a surgical insertion device according to this disclosure. For the purpose of teaching, electrodes (5) are inserted into a nerve bundle (31, 32, 33). The electrode can be formed on polymers. As illustrated in FIG. 2, these polymers are shaped in a saw tooth shaped profile near the sloped device edges to allow for stable fixation of the implanted device after insertion thereof in the tissue (31, 32, 33). FIG. 4a illustrates how the surgical insertion device (1), containing at one distal end the electrodes (5) connect to a device (10), positioned remote from these electrodes (5), is to be brought into contact with the tissue (31, 32, 33). A surgical needle holder (8) is used to manipulate the insertion device (1). FIG. 4b illustrates how the insertion device (3) is pushed into the tissue (31, 32, 33) with the sharp end first until its tip (3) becomes visible at the opposite side of the tissue (31, 32, 33). Then the tip can be taken by, e.g. a pair of tweezers, and by pulling at the tip, the insertion device (1) will slide further, well controlled, through the tissue (31, 32, 33), i.e. at the predetermined insertion position along the predetermined orientation. One continues pulling at the insertion device until the electrodes (5) are at the desired position with regard to the tissue (31, 32, 33). Once the electrodes (5) are at the desired position, it will remain fixed thanks to the saw tooth sides of the insertion device (1). Additionally, the part (4) of the insertion device (1) containing the electrodes (5) can be further fixed by a suture (9) through the tissue (31, 32, 33) and the fixation hole (6). The device (10) remains in electrical contact with the tissue (31, 32, 33) to-be-examined via the electrodes (5) which are in electrically connected to the device (10) via conductive lines on the insertion device (1).

## Claims

1. A surgical insertion device (1), comprising:
- an elongated central flat region (2) tapering from one distal end having a device area (4), configured to receive an electronic device (5), towards the opposite distal end having a sharp tip (3).

2. The insertion device (1) of claim 1, wherein:
- the central flat region (2), the sharp tip (3) and the device area (4) are integral with each other.

3. The insertion device (1) of claim 1, wherein:
- the sharp tip (3) is attached to the central flat region (2).

4. The insertion device (1) of any of the foregoing claims, further comprising:
- at least one opening (6) adjacent to the device area (4).

5. The insertion device (1) of any of the foregoing claims, further comprising:
- barbs (7) at least at one side near the maximal width (W2) of the insertion device (1).

6. The insertion device (1) of any of the foregoing claims, further comprising:
- an electronic device (5) attached to it at the distal end opposite to the sharp tip (3).

7. The insertion device (1) of claim 6, wherein:
- the electronic device (5) is embedded in the insertion device (1).

8. The insertion device (1) of claim 6, further comprising:
- at least one electrode (10), electrically connected to the electronic device (5), positioned nearer to the sharp tip (3).
